# EUROPÄISCHE PATENTSCHRIFT

(11) **EP 2 990 385 B1**
(45) Veröffentlichungstag und Bekanntmachung des Hinweises auf die Patenterteilung: **11.10.2017**
(21) Anmeldenummer: 15181291.4
(22) Anmeldetag: 17.08.2015
(51) Int. Cl.: C02F 1/44, C02F 1/26, C10L 9/08, C07C 51/47, C07D 307/48, B01D 61/14, C02F 103/36, C02F 101/34

(54) **VERFAHREN ZUR BEHANDLUNG VON PROZESSWASSER**
METHOD FOR TREATMENT OF PROCESS WATER
PROCÉDÉ DE TRAITEMENT D'EAU DE PROCESSUS

(30) Priorität: 26.08.2014 DE 102014112240
(43) Veröffentlichungstag der Anmeldung: 02.03.2016
(73) Patentinhaber: AVA-Co2 Schweiz AG, 6304 Zug (CH)
(72) Erfinder: Vyskocil, Jan, 6045 Meggen (CH); Koehler, Stephan, 78467 Konstanz (DE); Badoux, François, 6343 Rotkreuz (CH)
(74) Vertreter: Geitz Truckenmüller Lucht Christ

(56) Entgegenhaltungen:
- DE-A1-102009 027 007
- DE-A1-102011 053 034
- DE-A1-102012 002 590
- DE-U1-202012 100 129
- US-A- 5 635 071
- US-A1- 2008 237 110
- US-A1- 2012 042 566

## Beschreibung

Die vorliegende Erfindung betrifft ein Verfahren zur Behandlung von Prozesswasser, insbesondere im Vorfeld einer Extraktion der Reaktionsprodukte.

Der Stand der Technik kennt einige Verfahren zur Behandlung von Prozesswasser, so beispielsweise die DE 10 2012 002 590 A1 desselben Anmelders. Dort wird vorgeschlagen, im Rahmen eines hydrothermalen Karbonisierungsverfahren anfallendem Prozesswasser durch eine zweistufige Membranfilterung organische Substanzen zu entziehen, um diese dem Reaktionsprozess zu dessen Effizienzsteigerung wieder zuzuführen.

Die DE 10 2011 053 034 A1 desselben Anmelders offenbart ein Verfahren zur Gewinnung von Furfuralen, bei welchem mittels eines Trennverfahrens Hydroxymethylfurfural abgetrennt und aus dem Prozess ausgeschleust wird.

Aus der DE 20 2012 100 129 U1 desselben Anmelders ist es bekannt, mithilfe einer Filtervorrichtung Biokohle von dem Prozesswasser einer hydrothermalen

Karbonisierungsreaktion abzuscheiden, wobei der Filtrationsvorgang mittels Ultrafiltration durchgeführt wird.

Die US 5,635,071 A1 sieht vor, eine Abscheidung von Carboxylsäure mithilfe eines Membranfilters durchzuführen.

Im Rahmen der hydrothermalen Karbonisierung wird Biomasse in einem Reaktionsbehälter unter Zugabe von Dampf, in einem Temperaturbereich von etwa 150-230 °C und bei einem erhöhten Druck von etwa 4-28 bar karbonisiert. Neben der so entstehenden Biokohle fallen auch andere Produkte in diesem Prozess an, so insbesondere die Basischemikalien 5-Hydroxymethylfurfural, Furfural und Lävulinsäure. Diese werden in einem nachgelagerten Schritt aus dem Prozesswasser extrahiert, welches hierfür eine Extraktionskolonne durchläuft.

Neben der eigentlich gewünschten Karbonisierungsreaktion fallen jedoch vielfältige Nebenreaktionen an, so dass sich neben den gewünschten Produkten auch eine Reihe von Huminstoffen und Oligomeren bildet. Mit Oligomeren sind hierbei kleine, verzweigte Ketten aus einzelnen Monomeren der Produkte gemeint. Diese haben aufgrund ihrer Entstehung eine starke Ähnlichkeit hinsichtlich physikalischer und chemischer Eigenschaften zu den Produkten und lassen sich in den nachfolgenden Prozessschritten nicht bzw. nur sehr schwer von den Produkten trennen und führen somit zu einer Senkung der Produktreinheit. Ebenso wie die ebenfalls entstehenden Huminstoffe stellen die Oligomere also in der Extraktion Störstoffe dar, wobei deutlich mehr Huminstoffe anfallen. Die Huminstoffe können sowohl gelöst als auch ungelöst anfallen, wobei der ungelöste Teil durch Anlagerungen während der Abkühlung im Wärmetauscher zu Senkungen des Durchsatzes und Verunreinigungen führt, so dass teilweise der Herstellungsprozess der Produkte zur Reinigung des Wärmetauschers unterbrochen werden muss. Die gelösten Anteile hingegen stören die weiterführenden Prozessschritte der Produktisolierung, also insbesondere die Extraktion der Produkte. Sie lagern sich an den Wänden und Trennvorrichtungen der Extraktionskolonnen ab und erfordern hierdurch einen hohen Reinigungsaufwand, um die Anlagen in Betrieb zu halten.

Vor diesem Hintergrund liegt der vorliegenden Erfindung die Aufgabe zugrunde, den Reinigungsaufwand an den Extraktionskolonnen niedrig zu halten und gleichzeitig die Produktreinheit zu verbessern und damit die Qualität des Produktes, also des zu extrahierenden 5-Hydroxymethylfurfural, des Furfural und der Lävulinsäure, zu steigern.

Dies gelingt durch ein Verfahren zur Behandlung von Prozesswasser eines hydrothermalen Karbonisierungsprozesses gemäß den Merkmalen des Anspruchs 1. Weitere, sinnvolle Ausgestaltungen eines derartigen Verfahrens können den Unteransprüchen entnommen werden.

Erfindungsgemäß wird hierzu das Prozesswasser vor dem Eintritt in die Extraktionskolonne durch eine Filterkaskade hindurchgeleitet, welche mehrere Filtrationsstufen aufweist. Bei jeder Filtrationsstufe entfallen die Anteile in dem Prozesswasser, deren Größe der einzelnen Teilchen die Durchlässigkeit der jeweiligen Filtrationsstufe übersteigt. So werden die Trennmittel in der ersten Filtrationsstufe so gewählt, dass im Wesentlichen die Störstoffe, also die Huminstoffe und die Oligomere, als Retentat zurückgehalten werden. In dem den Hauptstrom darstellenden Permeat enthalten sind weiterhin die Produkte 5-Hydroxymethylfurfural, Furfural und Lävulinsäure, sowie die Edukte wie beispielsweise Fructose oder Glukose. Dies gilt insbesondere für flüssige Edukte. Soweit feste Edukte, wie beispielsweise Topinambur oder Holzchips, verwendet werden, so verbleiben diese im Reaktor.

In einer zweiten Filtrationsstufe, welcher das Permeat der ersten Filtrationsstufe als Zustrom zugeführt wird, ist wiederum das Trennmittel so gewählt, dass die Edukte, also im Wesentlichen Fructose und Glukose, als zweites Retentat zurückgehalten werden, während ein zweites Permeat mehr oder minder nur noch die Produkte 5-Hydroxymethylfurfural, Furfural und Lävulinsäure sowie alle Stoffe enthält, die wiederum kleiner sind als diese.

Durch diese Vorgehensweise werden gleich mehrere Probleme auf einmal gelöst. Durch die erste Filtration entfallen die Huminstoffe, welche die Extraktionskolonnen verschmutzen und daher zu großem Aufwand führen. Ebenfalls entfallen im Rahmen der ersten Filtration die Oligomere, welche das Produkt verunreinigen würden. Die im zweiten Schritt abgeschiedene Fructose bzw. Glukose kann dem Verfahren wieder zugeführt, also zurück in den Reaktor verbracht werden und dort die Ausbeute in einem weiteren Durchgang verbessern.

Mit einigem Vorteil kann eventuell durch weitere Filtrationsstufen eine weitere Auftrennung des zweiten Permeats erfolgen, so dass eine nacheinander erfolgende Filtrierung mit einem Trennmittel, dessen Durchlässigkeit einmal knapp über der Größe der Produkte und einmal knapp unter der Größe der Produkte liegt, ein exaktes Ausfiltern einzelner Produkte erfolgen. Maßgeblich ist hierbei jeweils die Molekülgröße.

Ebenso kann auch mit dem zweiten Retentat verfahren werden. Innerhalb dieser aufgezeigten Filtrationskaskade hat die letzte Trennstufe die Aufgabe inne, die Konzentration der Produkte bzw. der Edukte mittels Abführung von Reaktionswasser zu erhöhen. Durch diesen Schritt kann die nachfolgende Extraktion effizienter durchgeführt werden. Die entstandenen Permeate eignen sich besonders für deren Rückführung innerhalb der Kaskade an eine frühere Filtrationsstufe, so dass in diese keine zusätzlichen Stoffe, wie beispielsweise gelöste Salze, organische Stoffe oder Bakterien, in das System gebracht werden und damit den Reinheitsgrad wiederum erhöhen.

Als Trennmittel in den einzelnen Filtrationsstufen sind bevorzugtermaßen Membranfilter vorgesehen, besonders bevorzugt handelt es sich um LösungsDiffusionsmembranen und/oder ionenselektive Membranen wie Anionenaustauschermembranen oder Kationenaustauschermembranen, da hiermit die Ausbeute bei gleichzeitiger Reduzierung von Betriebs- und Hilfsstoffen verbessert werden kann.

Ferner hat es sich als sinnvoll erwiesen, den Zustrom von Prozesswasser und/oder Permeat zu einer Filtrationsstufe gleichmäßig zu halten, mithin dafür zu sorgen, dass der Zustrom dem Abstrom mehr oder weniger entspricht.

Nachdem das Prozesswasser, so wie es direkt aus dem Reaktor der hydrothermalen Karbonisierung entnommen wird, auch die entstandene Biokohle noch enthält, ist es zudem sinnvoll, dass Prozesswasser vor Eintritt in die erste Filtrationsstufe einer separaten Fest-Flüssig-Filtrationsstufe zuzuführen. Hierzu wird bevorzugtermaßen eine Oberflächen- oder Tiefenfiltration, besonders bevorzugt auch hier eine Membranfiltration durchgeführt.

Die vorstehend beschriebene Erfindung wird im Folgenden anhand eines Ausführungsbeispiels näher erläutert.

### Es zeigen

- Figur 1: eine einfache Filterkaskade mit zwei Filterstufen in einer schematischen Darstellung, und
- Figur 2: eine erweiterte Filterkaskade mit drei Filterstufen in einer schematischen Darstellung.

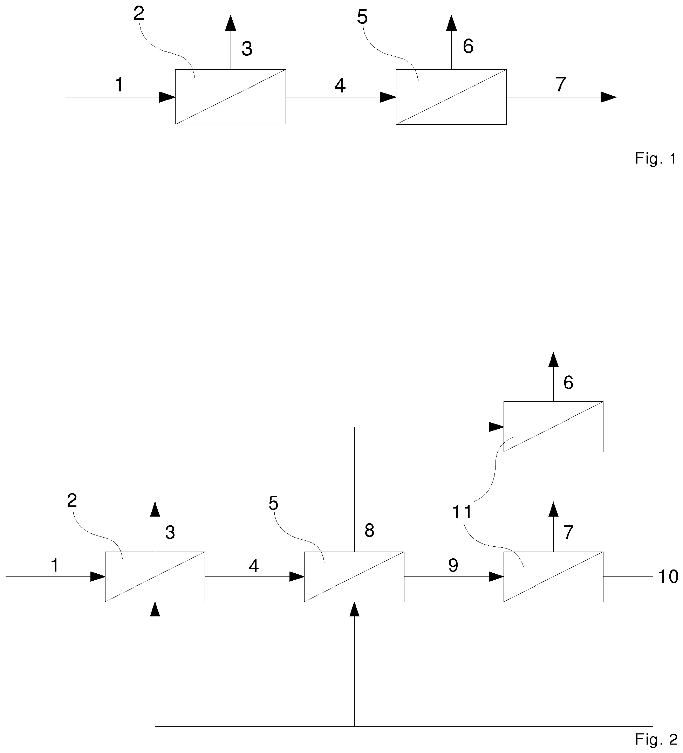

Figur 1 zeigt eine einfache Filterkaskade, mit welcher ankommendes Prozesswasser 1 aus dem hydrothermalen Karbonisierungsprozess vor dem Eintritt in die Extraktionskolonne vorbehandelt werden soll. Zu dem betrachteten Zeitpunkt ist das Prozesswasser 1 bereits durch eine Fest-Flüssig-Filtration, die hier nicht näher interessiert und daher nicht gezeichnet ist, hindurchgegangen und tritt in dieser Situation in eine erste Filtrationsstufe 2 ein. Es handelt sich hierbei um eine Membran-Filtrationsstufe, deren Durchlässigkeit derart gestaltet ist, dass Störstoffe 3 wie Huminstoffe oder Oligomere als Retentat in der ersten Filtrationsstufe 2 zurückgehalten werden, während Produkte 7 und Edukte 6, also 5-Hydroxymethylfurfural, Furfural und Lävulinsäure einerseits und Zucker, Zuckerderivate und hierbei insbesondere Fructose andererseits, in einem ersten Permeat 4 durch die erste Filtrationsstufe 2 hindurchgehen. Die Störstoffe 3 werden aus der ersten Filtrationsstufe 2 abgeführt und einer weiteren Verwertung oder Entsorgung zugeführt. Das erste Permeat 4, welches die Edukte 6 und die Produkte 7 enthält, wird sodann einer zweiten Filtrationsstufe 5 zugeführt, welche ebenfalls eine Membranfiltrationsstufe darstellt. Die Durchlässigkeit dieser zweiten Filtrationsstufe 5 ist jedoch so gewählt, dass die Edukte 6 zurückgehalten werden, während die Produkte 7 erneut durch die zweite Filtrationsstufe 5 hindurch treten können. In diesem Fall werden die Edukte 6 als Retentat zurückgehalten und beispielsweise wieder in den Prozess der hydrothermalen Karbonisierung zurückgeführt. Das zweite Permeat mit den Produkten 7 kann nunmehr der Extraktionskolonne zugeführt werden.

Figur 2 zeigt eine Alternative zu der in Figur 1 gezeigten Filterkaskade, bei welcher sowohl das zweite Retentat 8 als auch das zweite Permeat 9 einer erneuten, weiteren Filtrationsstufe 11 zugeführt werden. Durch die erneute Filtrierung erfolgt eine Aufkonzentrierung der gewünschten Stoffe so, dass die nachfolgenden Prozesse effektiver ablaufen, mithin im Hinblick auf die Produkte 7 die Extraktionskolonnen effektiver arbeiten können. Die weiteren Permeate 10 aus den weiteren Filtrationsstufen 11 werden hierbei zudem den früheren Filtrationsstufen, also der ersten Filtrationsstufe 2 und der zweiten Filtrationsstufe 5 zugeführt und bringen so einen Auswaschungseffekt als Unterstützung für den Trennvorgang mittels Diafiltration mit sich.

Vorstehend beschrieben ist somit ein Verfahren zur Behandlung von Prozesswasser eines hydrothermalen Karbonisierungsprozesses, in dessen Verlauf das im hydrothermalen Karbonisierungsprozess anfallende Prozesswasser derart filtriert wird, dass sich eine höhere Produktreinheit und gleichzeitig ein niedrigerer Verschmutzungsgrad für die betroffenen Anlagen einstellt und somit die Effizienz des Verfahrens deutlich verbessert wird.

## Patentansprüche

1. Verfahren zur Behandlung von Prozesswasser (1) eines hydrothermalen Karbonisierungsprozesses, wobei in einer ersten Filtrationsstufe (2) mittels eines Membranfilters das Prozesswasser (1) in ein im Wesentlichen Huminstoffe und/oder Oligomere enthaltendes erstes Retentat und ein im Wesentlichen Produkte (7), nämlich 5-Hydroxymethylfurfural und/oder Furfural und/oder Lävulinsäure, und Edukte (6), nämlich Glukose und/oder Fruktose, enthaltendes erstes Permeat (4) getrennt wird und das erste Permeat (4) einer zweiten Filtrationsstufe (5) mittels eines Membranfilters zugeführt wird, in der das erste Permeat (4) in ein im Wesentlichen Glukose und/oder Fruktose enthaltendes zweites Retentat (8) und ein im Wesentlichen 5-Hydroxymethylfurfural und/oder Furfural und/oder Lävulinsäure enthaltendes zweites Permeat (9) getrennt wird, wonach das zweite Permeat (9) einem Extraktionsschritt zur Extraktion der Produkte (7) mittels einer Extraktionskolonne zugeführt wird.

2. Verfahren gemäß Anspruch 1, **dadurch gekennzeichnet, dass** das zweite Permeat (9) vor der Zuführung zu dem Extraktionsschritt wenigstens einer weiteren Filtrationsstufe (11) zugeführt wird, in welcher das zweite Permeat (9) in unterschiedliche Einzelprodukte enthaltende weitere Permeate (10) und weitere Retentate getrennt wird.

3. Verfahren gemäß einem der Ansprüche 1 oder 2, **dadurch gekennzeichnet, dass** das zweite Retentat (8) in wenigstens einer weiteren Filtrationsstufe (11) aufkonzentriert wird.

4. Verfahren gemäß einem der vorhergehenden Ansprüche, **dadurch gekennzeichnet, dass** die Permeate (10) der letzten Filtrationsstufe oder Filtrationsstufen (11) früheren Filtrationsstufen (2, 5) als Zustrom zugeführt werden.

5. Verfahren gemäß einem der vorhergehenden Ansprüche, **dadurch gekennzeichnet, dass** sich die aufeinander folgenden Filtrationsstufen dadurch unterscheiden, dass sie Moleküle von zunehmend geringerer Größe zurückhalten.

6. Verfahren gemäß einem der vorhergehenden Ansprüche, **dadurch gekennzeichnet, dass** der Zustrom von Prozesswasser (1) und/oder Permeat (4, 9, 10) zu einer Filtrationsstufe (2, 5, 11) gleichmäßig erfolgt.

7. Verfahren gemäß Anspruch 6, **dadurch gekennzeichnet, dass** der Zustrom von Prozesswasser (1) und/oder Permeat (4, 9, 10) zu einer Filtrationsstufe (2, 5, 11) so eingestellt ist, dass die Abflussmenge der Zuflussmenge höchstens entspricht.

8. Verfahren gemäß einem der vorhergehenden Ansprüche, **dadurch gekennzeichnet, dass** das Prozesswasser (1) vor Eintritt in die erste Filtrationsstufe (2) einer separaten Fest-Flüssig-Filtrationsstufe zugeführt wird.

## Claims

1. A method for the treatment of process water (1) of a hydrothermal carbonization process, wherein, in a first filtration stage (2), the process water (1) is separated by means of a membrane filter into a first retentate containing essentially humic substances and/or oligomers and a first permeate (4) essentially containing products (7), namely 5-hydroxymethylfurfural and/or furfural and/or levulinic acid, and educts (6), namely glucose and/or fructose (9), and the first permeate (4) is fed to a second filtration stage (5) by means of a membrane filter, in which the first permeate (4) is separated into a second retentate (8) containing essentially glucose and/or fructose and a second permeate (9) containing essentially 5-hydroxymethylfurfural and/or furfural and/or levulinic acid, whereupon the second permeate (9) is fed to an extraction step for the extraction of the products (7) by means of an extraction column.

2. A method according to claim 1, **characterized in that** the second permeate (9) is fed to at least one further filtration stage (11) before the feed to the extraction step, in which the second permeate (9) is separated into further permeates (10), which contain different individual products, and further retentates.

3. A method according to one of the claims 1 or 2, **characterized in that** the second retentate (8) is concentrated in at least one further filtration stage (11).

4. A method according to one of the preceding claims, **characterized in that** the permeates (10) of the last filtration stage or filtration stages (11) are fed to previous filtration stages (2, 5) as a feed stream.

5. A method according to one of the preceding claims, **characterized in that** the successive filtration stages differ **in that** they retain molecules of increasingly smaller size.

6. A method according to one of the preceding claims, **characterized in that** the feed stream of process water (1) and/or permeate (4, 9, 10) to a filtration stage (2, 5, 11) occurs uniformly.

7. A method according to claim 6, **characterized in that** the feed stream of process water (1) and/or permeate (4, 9, 10) to a filtration stage (2, 5, 11) is set such that the outflow quantity corresponds at most to the inflow quantity.

8. A method according to one of the preceding claims, **characterized in that** the process water (1) is fed to a separate solid-liquid filtration stage before entering the first filtration stage (2).

## Revendications

1. Procédé de traitement d'une eau de processus (1) d'un processus de carbonatation hydrothermale, dans lequel, dans un premier étage de filtration (2) au moyen d'un filtre à membrane, l'eau de processus (1) est séparée en un premier rétentat contenant sensiblement des humines et/ou des oligomères et un premier perméat (4) contenant sensiblement des produits (7), à savoir du 5-hydroxyméthylfurfural et/ou du furfural et/ou de l'acide lévulinique, et des éduits (6), à savoir du glucose et/ou du fructose, et le premier perméat (4) est amené à un deuxième étage de filtration (5) au moyen d'un filtre à membrane, dans lequel le premier perméat (4) est séparé en un deuxième rétentat (8) contenant sensiblement du glucose et/ou du fructose et un deuxième perméat (9) contenant sensiblement du 5-hydroxyméthylfurfural et/ou du furfural et/ou de l'acide lévulinique, après quoi le deuxième perméat (9) est amené à une étape d'extraction pour l'extraction des produits (7) au moyen d'une colonne d'extraction.

2. Procédé selon la revendication 1, **caractérisé en ce que** le deuxième perméat (9), avant d'être amené à l'étape d'extraction, est amené à au moins un autre étage de filtration (11) dans lequel le deuxième perméat (9) est séparé en d'autres perméats (10) contenant différents produits individuels et en d'autres rétentats.

3. Procédé selon l'une des revendications 1 ou 2, **caractérisé en ce que** le deuxième rétentat (8) est concentré dans au moins un autre étage de filtration (11).

4. Procédé selon l'une des revendications précédentes, **caractérisé en ce que** les perméats (10) du ou des dernier(s) étage(s) de filtration (11) sont amenés en tant que flux entrant aux étages de filtration précédents (2, 5).

5. Procédé selon l'une des revendications précédentes, **caractérisé en ce que** les étages de filtration successifs diffèrent **en ce qu'**ils retiennent des molécules de taille de plus en plus faible.

6. Procédé selon l'une des revendications précédentes, **caractérisé en ce que** l'amenée d'eau de processus (1) et/ou de perméat (4, 9, 10) à un étage de filtration (2, 5, 11) a lieu de manière uniforme.

7. Procédé selon la revendication 6, **caractérisé en ce que** l'amenée d'eau de processus (1) et/ou de perméat (4, 9, 10) à un étage de filtration (2, 5, 11) est réglée de façon que le débit sortant corresponde au maximum au débit entrant.

8. Procédé selon l'une des revendications précédentes, **caractérisé en ce qu'**avant l'entrée dans le premier étage de filtration (2), l'eau de processus (1) est amenée à un étage de filtration solide-liquide séparé.
